# EUROPEAN PATENT APPLICATION

(11) **EP 4 595 890 A1**
(43) Date of publication of application: **06.08.2025**
(21) Application number: 25153939.1
(22) Date of filing: 24.01.2025
(51) Int. Cl.: A61B 6/03, A61B 6/04, A61B 6/00

(54) **INFORMATION PROCESSING APPARATUS, MEDICAL IMAGE CAPTURING APPARATUS, INFORMATION PROCESSING METHOD, AND PROGRAM**

(30) Priority: 01.02.2024 JP 2024014172
(71) Applicant: FUJIFILM Corporation, Tokyo Tokyo 106-8620 (JP)
(72) Inventor: FUJITA, Mitsuyo, Tokyo (JP); HAYASHI, Tetsuya, Tokyo (JP)
(74) Representative: Dehns Germany Partnerschaft mbB

(57) **Abstract**

Provided are an information processing apparatus, a medical image capturing apparatus, an information processing method, and a program capable of suppressing display shake caused by display of a line indicating a capturing range of scanogram capturing, as compared with a technology of the related art.

An information processing apparatus (10) includes a display controller that continuously acquires image information obtained by optically capturing a subject in a decubitus state on a bed while performing control to display a first line in a case where a difference between a position of the first line corresponding to a position of a predetermined part of the subject in an image indicated by the image information at a current point in time and a position of a second line corresponding to a position of the above-described predetermined part of the subject in an image indicated by the image information in a most recent past exceeds a predetermined threshold value.

## Description

### BACKGROUND OF THE INVENTION

### 1. Field of the Invention

The present disclosure relates to an information processing apparatus, a medical image capturing apparatus, an information processing method, and a program.

### 2. Description of the Related Art

In recent years, with the advancement of medical equipment, such as a computed tomography (CT) device and a magnetic resonance imaging (MRI) device, higher quality and high-resolution three-dimensional images have been used for image diagnosis.

In a case where a capturing apparatus such as a CT device and an MRI device captures a subject, in order to determine a capturing range, a scanogram is captured (hereinafter, referred to as "scanogram capturing") before main capturing for acquiring a three-dimensional image, and a two-dimensional positioning image (scanogram) is acquired. An operator (technician or the like) of the capturing apparatus sets a capturing range at a time of the main capturing while viewing the scanogram. The scanogram is also referred to as a scout image, a topogram, or the like, but will be referred to as a "scanogram" in the following.

Before the scanogram capturing, the operator sets a capturing range of the scanogram capturing in the subject on a bed. For example, a cross-shaped laser is emitted to the subject, a scanogram start position of the scanogram capturing is set, and a scanogram end position of the scanogram capturing is set to be a capturing range in accordance with a capturing part. During the scanogram capturing, in a case where the bed is moved from an initial position of the bed to the scanogram start position, the scanogram capturing is started, and in a case where the bed is moved to the scanogram end position, the scanogram capturing is ended. The operator sets a capturing range of the main capturing by the scanogram acquired by the scanogram capturing, and then performs the main capturing to acquire the three-dimensional image.

Here, in a case of setting the capturing range during the scanogram capturing, the subject is captured by a camera provided above the bed, feature points such as both ankles, both waists, both elbows, and both shoulders of the subject are detected, and the capturing range is specified based on the detected feature points. In this case, a trained detection model constructed by machine learning a neural network is used for the detection of the feature points.

As a technology related to capturing by a medical capturing apparatus as described above, JP2021-062126A discloses a medical image diagnosis apparatus for a purpose of making the setting of a capturing cross section position or a capturing range more efficient than that in the related art.

The medical image diagnosis apparatus comprises a stand device having a capturing system that captures a subject using radiation or magnetism and an image generation unit that generates a first image obtained by capturing the subject with an optical capturing device different from the capturing system and a second image in which planes related to a capturing position in the capturing using the capturing system are synthesized.

### SUMMARY OF THE INVENTION

By the way, in the related art, in a case of performing scanogram capturing, there is a technology of specifying a capturing range of the scanogram capturing based on a position of a subject image obtained by optically capturing the subject in a decubitus state on a bed and of displaying a line indicating the capturing range in real time by superimposing the line on the above-described subject image, a schema, or the like. With reference to this display, an operator can check the capturing range of the scanogram capturing in a state of following movement of the subject before the scanogram capturing.

However, in this technology, since the line indicating the above-described capturing range is displayed by being superimposed on another image in real time, there is a problem that a shake (hereinafter, referred to as a "display shake") is likely to occur in a display image. In the technology disclosed in JP2021-062126A, the display shake is not considered, and this problem cannot be solved.

The present disclosure has been made in view of the above-described circumstances, and an object of the present disclosure is to provide an information processing apparatus, a medical image capturing apparatus, an information processing method, and a program capable of suppressing display shake caused by display of a line indicating a capturing range of scanogram capturing, as compared with a technology of the related art.

In order to achieve the above-described object, according to a first aspect of the present disclosure, an information processing apparatus comprises at least one processor, in which the processor is configured to: continuously acquire image information obtained by optically capturing a subject in a decubitus state on a bed; and perform control to display a first line in a case where a difference between a position of the first line corresponding to a position of a predetermined part of the subject in an image indicated by the image information at a current point in time and a position of a second line corresponding to a position of the above-described predetermined part of the subject in an image indicated by the image information in a most recent past exceeds a predetermined threshold value.

According to a second embodiment, in the information processing apparatus according to the first aspect, the processor is configured to perform control to display the second line in a case where the difference is equal to or less than the threshold value.

According to a third embodiment, in the information processing apparatus according to the first aspect or the second embodiment, the position of the second line is a position obtained by a moving average using images indicated by a plurality of pieces of the image information in the most recent past.

According to a fourth embodiment, in the information processing apparatus according to the first aspect or the second embodiment, the position of the second line is a position obtained by an image indicated by a single piece of the image information in the most recent past.

According to a fifth embodiment, in the information processing apparatus according to the first aspect, the processor is configured to perform control using the difference in a case where a distance between the bed and a capturing apparatus that performs the optical capturing changes exceeding a predetermined distance due to movement of the bed, in a case where the bed is movable in an up-down direction and the capturing apparatus is fixed above the bed.

According to a sixth embodiment, in the information processing apparatus according to the fifth embodiment, the processor changes the threshold value in accordance with the distance.

On the other hand, in order to achieve the above-described object, there is provided a medical image capturing apparatus according to a another aspect of the present invention comprises the information processing apparatus according to the present invention, and a radiography apparatus controlled by the information processing apparatus.

In addition, in order to achieve the above-described object, according to another aspect of the present invention there is provided an information processing method causes a computer to execute: continuously acquiring image information obtained by optically capturing a subject in a decubitus state on a bed; and performing control to display a first line in a case where a difference between a position of the first line corresponding to a position of a predetermined part of the subject in an image indicated by the image information at a current point in time and a position of a second line corresponding to a position of the above-described predetermined part of the subject in an image indicated by the image information in a most recent past exceeds a predetermined threshold value.

Further, in order to achieve the above-described object, according to another aspect of the present disclosure, a program causes a computer to execute a process including continuously acquiring image information obtained by optically capturing a subject in a decubitus state on a bed; and performing control to display a first line in a case where a difference between a position of the first line corresponding to a position of a predetermined part of the subject in an image indicated by the image information at a current point in time and a position of a second line corresponding to a position of the above-described predetermined part of the subject in an image indicated by the image information in a most recent past exceeds a predetermined threshold value.

According to the present disclosure, it is possible to suppress display shake caused by display of the line indicating the capturing range of the scanogram capturing, as compared with a technology of the related art.

### BRIEF DESCRIPTION OF THE DRAWINGS

Fig. 1 is a perspective view showing an outline of a CT device to which an information processing apparatus according to an embodiment is applied.
Fig. 2 is a diagram showing the CT device to which the information processing apparatus according to the embodiment is applied, as viewed from a side.
Fig. 3 is a block diagram showing a schematic configuration of the information processing apparatus according to the embodiment.
Fig. 4 is a block diagram showing a functional configuration of the information processing apparatus according to the embodiment.
Fig. 5 is a diagram for describing feature points according to the embodiment.
Fig. 6 is a diagram showing an example of a line display screen according to the embodiment.
Fig. 7 is a flowchart showing an example of a flow of information processing according to a first embodiment.
Fig. 8 is a diagram for describing processing according to a second embodiment and is a side view showing an example of a state in a case where a bed is moved up and down.
Fig. 9 is a diagram for describing the processing according to the second embodiment and is a diagram showing an example of a state of the line display screen in a case where the bed is moved up and down.
Fig. 10 is a flowchart showing an example of a flow of information processing according to the second embodiment.
Fig. 11 is a flowchart showing an example of a flow of second information processing according to the second embodiment.
Fig. 12 is a flowchart showing an example of a flow of third information processing according to the second embodiment.
Fig. 13 is a diagram showing an example of a line display screen according to another embodiment.
Fig. 14 is a diagram showing an example of the line display screen according to another embodiment.

### DESCRIPTION OF THE PREFERRED EMBODIMENTS

Hereinafter, a description of embodiments of the present disclosure will be made with reference to the accompanying drawings.

### First Embodiment

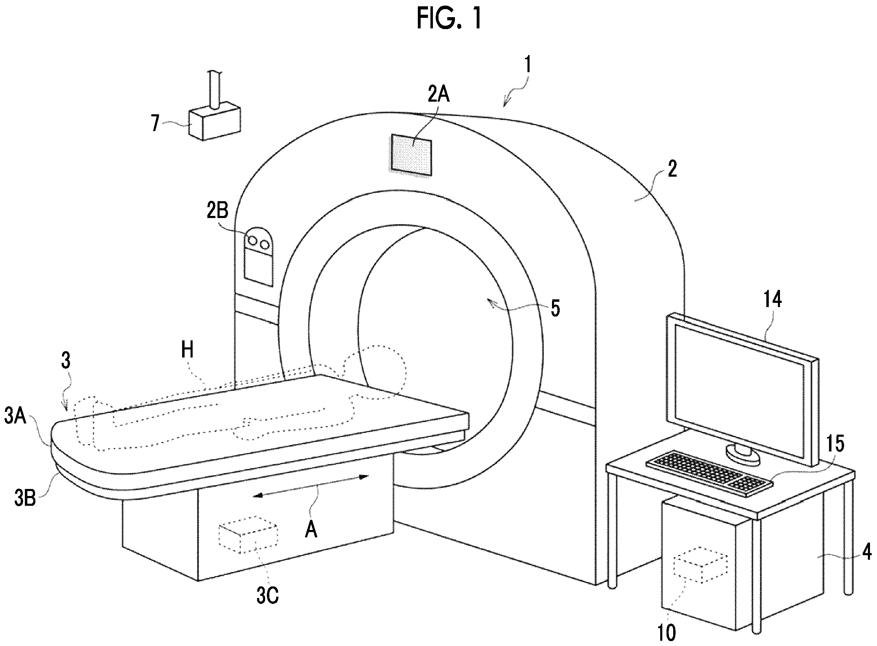

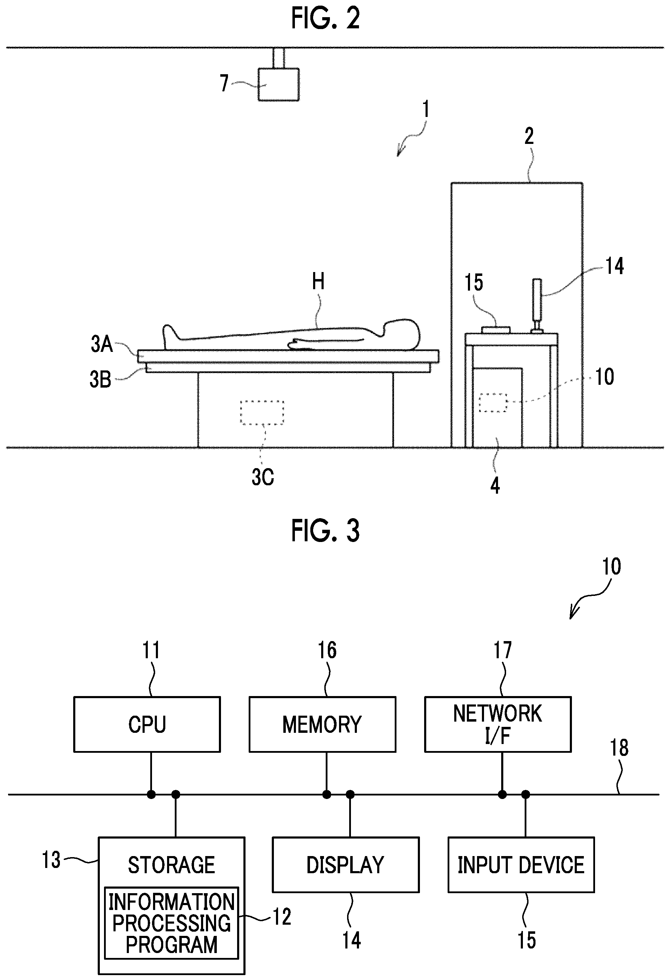

Fig. 1 is a perspective view showing an outline of a CT device to which an information processing apparatus according to an embodiment of the present disclosure is applied, and Fig. 2 is a diagram showing the CT device to which the information processing apparatus according to the embodiment of the present disclosure is applied, as viewed from the side.

As shown in Figs. 1 and 2, a CT device 1 according to the present embodiment comprises a gantry 2, a bed 3, and a console 4. A combination of the gantry 2 and the bed 3 corresponds to a radiography apparatus of the present disclosure, and a combination of the gantry 2, the bed 3, and the console 4 corresponds to the medical image capturing apparatus of the present disclosure.

The gantry 2 has a tunnel-like structure having an opening portion 5 at the center thereof. A radiation source unit that emits X-rays and a detection unit that detects X-rays and generates a radiation image are provided inside the gantry 2 (both not shown). Each of the radiation source unit and the detection unit can be rotated along an annular shape of the gantry 2 in a state in which a positional relationship of facing each other is maintained. A control unit that controls an operation of the CT device 1 is provided inside the gantry 2.

In addition, a display device 2A is provided on a front surface side (a side on which the bed 3 is disposed) and in a center portion of an upper portion of the gantry 2, and an operation button 2B is provided on a left side in a front view on the front surface side. The display device 2A according to the present embodiment can display various types of information including a line display screen described in detail below, under a control of the console 4. In addition, the operation button 2B according to the present embodiment is a button subjected to pressing operation by an operator in a case where a positioning of a subject with respect to the bed 3 is ended and scanogram capturing that acquires a two-dimensional image or a main capturing that acquires a three-dimensional image is performed.

On the other hand, the bed 3 includes a bed portion 3A on which the subject lies, a base portion 3B that supports the bed portion 3A, and a driving unit 3C that reciprocally moves the bed portion 3A in an arrow A direction. The bed portion 3A can slide with respect to the base portion 3B in the arrow A direction by the driving unit 3C. In a case where a CT image is captured, the bed portion 3A slides, so that a subject H lying on the bed portion 3A is transported into the opening portion 5 of the gantry 2.

A camera 7 is installed above the bed 3. The camera 7 is a camera that can capture a color image of red (R), green (G), and blue (B) by detecting reflected light of the subject H. The camera 7 has an imaging element such as a lens and a charge coupled device (CCD), acquires a camera image, which is a moving image, by capturing the subject H on the bed 3 at a predetermined frame rate, and outputs the camera image to the console 4. The camera 7 may be a camera in which an RGB camera and a near infrared (NIR) camera are integrated. In this case, the NIR camera is a stereo camera, and information in a depth direction of the subject H can be acquired by a parallax. By using the NIR camera of the camera 7 as the stereo camera, information in the depth direction of the subject H on the bed 3 can be acquired. The NIR camera can perform capturing even in a case where an amount of light is insufficient. Therefore, in a case where a brightness of an examination room is insufficient for the capturing of the RGB camera, the subject H may be captured by the NIR camera.

Driving of the gantry 2, driving of the bed 3, and capturing of the subject by the camera 7 are performed by an input of the operator from the console 4. The console 4 encompasses the information processing apparatus according to the present embodiment.

Next, the information processing apparatus according to the present embodiment encompassed in the console 4 will be described. First, with reference to Fig. 3, a hardware configuration of an information processing apparatus according to the present embodiment will be described.

As shown in Fig. 3, an information processing apparatus 10 is a computer, such as a workstation, a server computer, and a personal computer, and comprises a central processing unit (CPU) 11, a non-volatile storage 13, and a memory 16 as a transitory storage region. In addition, the information processing apparatus 10 comprises a display 14 such as a liquid crystal display, an input device 15 such as a keyboard and a mouse, and a network interface (I/F) 17 connected to the CT device 1. The CPU 11, the storage 13, the display 14, the input device 15, the memory 16, and the network I/F 17 are connected to a bus 18. Note that the CPU 11 is an example of a processor according to the present disclosure. The display 14 and the input device 15 are also shown in Figs. 1 and 2.

The storage 13 is realized by a hard disk drive (HDD), a solid state drive (SSD), a flash memory, and the like. The storage 13 as a storage medium stores an information processing program 12 installed in the information processing apparatus 10. The CPU 11 reads out the information processing program 12 from the storage 13, loads the information processing program 12 into the memory 16, and executes the loaded information processing program 12. A method of processing executed by the information processing program 12 corresponds to an information processing method of the present disclosure.

The information processing program 12 is stored in a storage device of a server computer connected to a network or in a network storage in a state of being accessible from the outside, and is downloaded and installed in a computer that configures the information processing apparatus 10 in response to a request. Alternatively, the information processing program 12 is recorded on a recording medium, such as a digital versatile disc (DVD) or a compact disc read only memory (CD-ROM), is distributed, and is installed in the computer that configures the information processing apparatus 10 from the recording medium.

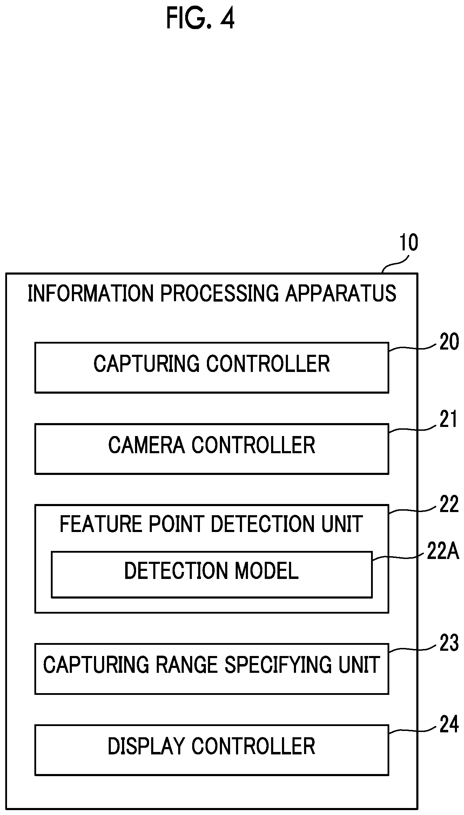

Next, a functional configuration of the information processing apparatus according to the present embodiment will be described. Fig. 4 is a diagram showing a functional configuration of the information processing apparatus according to the present embodiment.

As shown in Fig. 4, the information processing apparatus 10 comprises a capturing controller 20, a camera controller 21, a feature point detection unit 22, a capturing range specifying unit 23, and a display controller 24. Then, the CPU 11 functions as the capturing controller 20, the camera controller 21, the feature point detection unit 22, the capturing range specifying unit 23, and the display controller 24 by executing the information processing program 12.

The capturing controller 20 controls a capturing unit, a detection unit, and a controller provided in the gantry 2 such that capturing of the subject H is performed by an instruction from the input device 15. In a case of CT capturing, scanogram capturing is performed before main capturing that acquires a three-dimensional CT image in order to determine a capturing range. The scanogram capturing is performed by capturing the subject H in a state in which the capturing unit and the detection unit are fixed.

In a case of the scanogram capturing, the capturing range of the subject H is set, and the scanogram capturing is performed by moving the bed 3 to the opening portion 5 of the gantry 2 such that the set capturing range is captured. A scanogram acquired by the scanogram capturing is a two-dimensional image including the capturing range set for the subject H. The scanogram is displayed on the display 14. The operator sets the capturing range in a case of performing the main capturing by looking at the scanogram displayed on the display 14. After the capturing range is set, the operator performs pressing operation of the operation button 2B to give an instruction for the main capturing, so that the main capturing is performed and the three-dimensional CT image of the subject H is acquired. The acquired scanogram and CT image are stored in the storage 13.

The camera controller 21 controls the capturing of the subject H on the bed 3 by the camera 7. The capturing of the subject H by the camera 7 is performed in order to set the capturing range in a case of performing the scanogram capturing. The capturing of the subject H by the camera 7 is performed from a preparation stage before the capturing. That is, the camera controller 21 starts capturing by the camera 7 from a point in time before the subject H lies down on the bed 3 and causes the camera 7 to acquire the camera image. Then, in a case where an instruction to start the scanogram capturing is performed by the operator, the camera controller 21 stops the capturing by the camera 7. The acquired camera image is stored in the memory 16 in order to specify the capturing range described below.

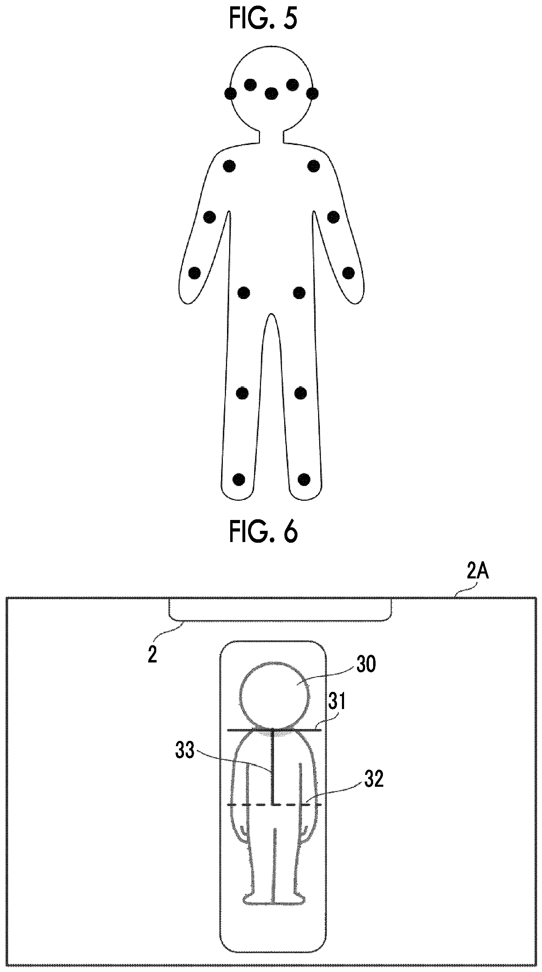

The feature point detection unit 22 detects a plurality of feature points on the subject H included in the camera image by using a detection model 22A constructed in advance. Fig. 5 is a diagram for describing feature points according to the present embodiment. As shown in Fig. 5, in the present embodiment, the detection model 22A is constructed to detect 17 feature points on the subject H included in the camera image. The 17 feature points are both eyes, a nose, both ears, both shoulders, both elbows, both hands, both waists, both knees, and both feet. 18 feature points may be used by adding a point in the middle of a collarbone to these 17 feature points.

The detection model 22A according to the present embodiment is constructed by performing machine learning on a neural network. In this neural network, a training image including an entire human body and having 17 known feature points, which is acquired by capturing the human body with the camera 7, is used for learning. The training image is acquired by capturing a person wearing an examination suit with the camera 7, for example, as in a case where an actual examination is performed.

The detection model 22A according to the present embodiment derives a probability representing a possibility of each of 17 feature points in each pixel of the camera image. Then, the feature point detection unit 22 detects, as the feature point, the pixel from which a highest probability is derived for each of the 17 feature points. For example, in a case where a right eye is detected as the feature point, the feature point detection unit 22 compares probabilities in which all pixels of the camera image derived by the detection model 22A are the right eye, and detects a pixel having the highest probability as the feature point of the right eye.

The capturing range specifying unit 23 specifies the capturing range of the subject H in a case of performing the scanogram capturing based on the 17 feature points detected by the feature point detection unit 22. For this purpose, the capturing range specifying unit 23 first determines detection accuracy of the feature point detected by the feature point detection unit 22. As described above, the feature point detection unit 22 detects, as the feature point, the pixel from which the highest probability is derived for each of the 17 feature points. As the probability output by the detection model 22A for the detected feature point is higher, the detection accuracy is better. Therefore, the capturing range specifying unit 23 compares a representative value of the probabilities derived by the detection model 22A for the 17 feature points with a predetermined threshold value, and determines that the detection accuracy is good in a case where the representative value is equal to or greater than the threshold value and that the detection accuracy is poor in a case where the representative value is less than the threshold value. As the representative value, an average value, a median value, a weighted average value in accordance with a capturing part, or the like can be used, but the present invention is not limited thereto.

In a case where it is determined that the detection accuracy is good, the capturing range specifying unit 23 performs processing of specifying the capturing range. On the other hand, in a case where it is determined that the detection accuracy is poor, the capturing range specifying unit 23 performs a warning display on the display 14. Here, in a case where the subject H moves too much, a thick blanket is placed on the subject H, or the entire body of the subject H is not included in the capturing range of the camera 7, the feature point cannot be detected with high accuracy even by using the detection model 22A, and the detection accuracy deteriorates. In such a case, the capturing range specifying unit 23 determines that the detection accuracy is poor.

In a case where the warning display is performed, the operator manually sets the capturing range of the scanogram capturing. That is, the operator measures a distance from the initial position of the bed to a scanogram start line described below, further measures a distance between the scanogram start line and a scanogram end line described below, and inputs the measured distance from the input device 15. The movement of the bed 3 during the scanogram capturing is controlled based on the input distance.

The capturing part of the subject H is included in an examination order provided by the doctor at a time of capturing, and the operator sets the capturing part using the input device 15 with reference to the examination order. The capturing parts of the subject H include a head, a chest, an abdomen, a lower limb, an entire body, and the like.

Hereinafter, processing of specifying the capturing range by the capturing range specifying unit 23 will be described. For example, in a case where the capturing part is a chest, the scanogram has a capturing range from a tip of a chin to a middle part of the abdomen. Therefore, the capturing range specifying unit 23 sets, among the feature points detected by the feature point detection unit 22, a line connecting both shoulders as the scanogram start line and a center line between a line connecting both elbows and a line connecting both hands is set as the scanogram end line. Then, the capturing range specifying unit 23 derives a distance D1 between the scanogram start line and the scanogram end line. A range of a distance D1 between the scanogram start line and the scanogram end line is the capturing range.

Here, before the scanogram capturing, the bed 3 is at the initial position, and the subject H is lying on the bed 3, so that a distance from an end portion of the bed 3 to the scanogram start line can be obtained, from the camera image. Therefore, the capturing range specifying unit 23 calculates a distance D2 from the end portion of the bed 3 to the scanogram start line as an amount of movement of the bed from the initial position to the scanogram start line, that is, an amount of movement of the bed 3 until the scanogram start line reaches the scanogram start position in the CT device 1.

In a case where the capturing range specifying unit 23 specifies the capturing range in the scanogram capturing, the display controller 24 performs control to display, in real time, an image in which the scanogram start line, the scanogram end line, and a midline connecting a center point of each of the scanogram start line and the scanogram end line are superimposed on the camera image on the display device 2A. Hereinafter, a screen displayed on the display device 2A in this case is referred to as a "line display screen".

Fig. 6 is a diagram showing an example of a line display screen according to the embodiment. As shown in Fig. 6, in the line display screen according to the present embodiment, a scanogram start line 31, a scanogram end line 32, and a midline 33 are shown together with an image (hereinafter, referred to as a "subject image") 30 of the subject H. The operator checks the scanogram start line 31, the scanogram end line 32, and the midline 33 displayed on the display device 2A. After the check, in a case where there is no problem, the operator performs the pressing operation of the operation button 2B, so that the instruction to start the scanogram capturing is performed.

By the instruction to start the scanogram capturing, information on the distances D1 and D2 is output to the CT device 1. The capturing controller 20 controls the CT device 1 such that the scanogram capturing starts after the driving unit 3C moves the bed 3 by the distance D2 and the scanogram capturing ends in a case where the driving unit 3C moves the bed 3 by the distance D1.

The scanogram acquired by the scanogram capturing is displayed on the display 14. The operator checks the scanogram displayed on the display 14 and sets the capturing range of the main capturing with respect to the scanogram. Then, the main capturing is performed by performing the pressing operation of the operation button 2B to perform a capturing instruction for the main capturing, and the three-dimensional CT image of the capturing part of the subject H is acquired in the set capturing range of the main capturing.

As described above, in the scanogram capturing according to the present embodiment, the display of the line display screen is performed in real time before the capturing is performed. In this case, each line indicating the capturing range is displayed in real time by being superimposed on the camera image in capturing frame units. Therefore, there is a problem that the display shake is likely to occur on the line display screen displayed on the display device 2A.

Therefore, in image information indicating the camera image acquired by the camera 7, in a case where a difference D between a position of a first line corresponding to a position of a predetermined part of the subject H in a camera image indicated by image information at a current point in time and a position of a second line corresponding to a position of the above-described predetermined part of the subject H in an image indicated by image information of a most recent past (in the present embodiment, one frame before the current point in time) exceeds a predetermined threshold value, the display controller 24 according to the present embodiment performs control to display the first line. On the other hand, in a case where the above-described difference D is equal to or less than the above-described threshold value, the display controller 24 according to the present embodiment performs control to display the second line.

In the present embodiment, as the first line and the second line, each line of the above-described scanogram start line 31, scanogram end line 32, and midline 33 (hereinafter, also simply referred to as a "line") is applied. Here, a difference between the first line and the second line is that the first line represents a line at a current point in time and the second line represents a line in the most recent past.

That is, in the display controller 24 according to the present embodiment, a deviation amount between the position of the line indicating the capturing range of the scanogram capturing in the current point in time and the position of the line indicating the capturing range of the scanogram capturing in the most recent past is derived as the difference D. Then, in a case where the derived difference D is equal to or greater than the above-described threshold value, the display controller 24 performs control to display the line at a current point in time, in other words, the latest line on the line display screen instead of the previous line.

On the other hand, in a case where the derived difference D is less than the above-described threshold value, the display controller 24 performs control to display the line in the most recent past, in other words, the line displayed so far, on the line display screen. Therefore, with the display controller 24 according to the present embodiment, in a case where the difference D, that is, the deviation amount from the most recent past of the capturing range of the scanogram capturing is small, the line to be displayed on the line display screen is not changed. Therefore, it is possible to suppress occurrence of the display shake of the line display screen described above. In the display of the line in the most recent past performed here, it is not always necessary to display the line again, and in a case where a display state of the line displayed so far is maintained in a visible manner on the line display screen, it is not always necessary to perform control to display the line again.

In the present embodiment, a largest distance among distances between scanogram start lines 31, distances between scanogram end lines 32, and distances between midlines 33, in the first line and the second line, is applied as the difference D. However, the present invention is not limited thereto. For example, a form may be adopted in which a smallest distance or an intermediate distance among the distances between the scanogram start lines 31, the distances between the scanogram end lines 32, and the distances between the midlines 33, in the first line and the second line, is applied as the difference D. In addition, in the present embodiment, a distance between center points of corresponding lines is applied as a distance between corresponding lines here, but the present invention is not limited thereto. For example, a form may be adopted in which a farthest distance between the corresponding lines is applied as the distance between the corresponding lines.

In addition, in the present embodiment, in a case where the difference D exceeds the value, as long as the display of the capturing range of the scanogram capturing on the line display screen is not updated, a value set in fixed manner as a value at which a problem occurs, is applied as the above-described threshold value, but the present invention is not limited thereto. For example, a form may be adopted in which the operator is caused to appropriately set the above-described threshold value in accordance with the accuracy required for the scanogram capturing, a behavior of the subject H, and the like.

Further, in the present embodiment, a position obtained by an image indicated by a single piece of the image information in the most recent past is applied as the position of the second line. Accordingly, processing time can be set to be shorter and a storage capacity for storing the image information can be set to be smaller than those in a case where the position obtained by a moving average using images indicated by a plurality of pieces of image information in the most recent past is applied as the position of the second line.

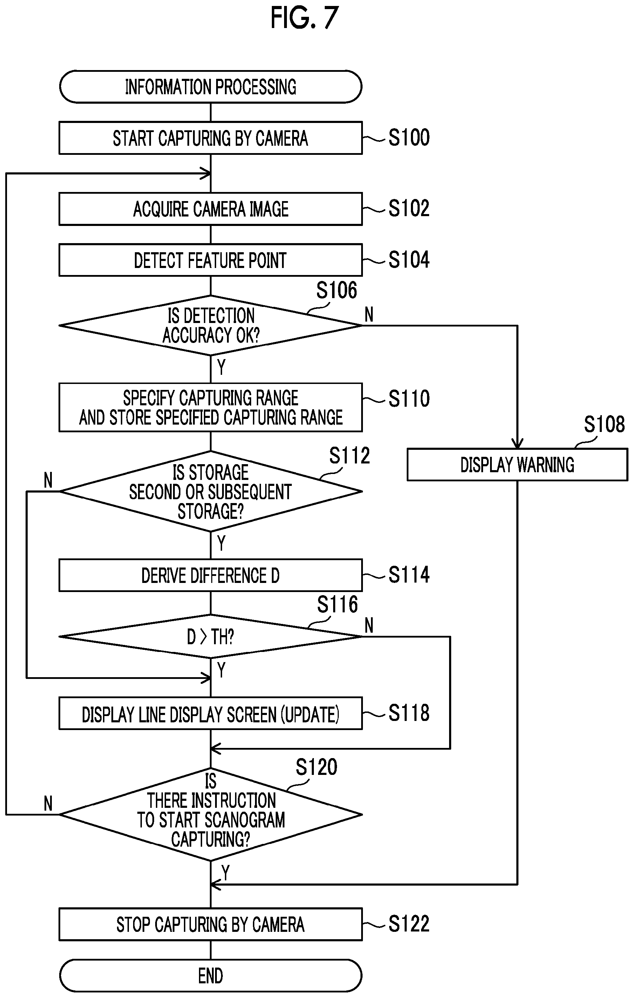

Next, information processing performed in a first embodiment will be described. Fig. 7 is a flowchart showing a flow of the information processing performed in the first embodiment. The information processing is started by performing an instruction to start capturing from the input device 15.

In step S100, the CPU 11 performs control to start the capturing by the camera 7. In step S102, the CPU 11 acquires a camera image of one frame. In step S104, the CPU 11 detects a feature point from the camera image using the detection model 22A.

In step S106, the CPU 11 determines whether or not the detection accuracy of the feature point is good, proceeds to step S108 in a case where a negative determination is made, the CPU 11 performs the warning display, and then proceeds to step S122. In a case where the warning display is performed, the operator manually sets the capturing range of the scanogram capturing as described above.

On the other hand, in a case where a positive determination is made in step S106, the process proceeds to step S110, the CPU 11 specifies the capturing range in the scanogram capturing and stores the specified capturing range in the memory 16, in step S112, the CPU 11 determines whether or not the storage is a second or subsequent storage, and in a case where a negative determination is made, the process proceeds to step S118, while in a case where the positive determination is made, the process proceeds to step S114.

In step S114, the CPU 11 derives the above-described difference D, and in step S116, the CPU 11 determines whether or not the difference D exceeds a threshold value TH. In a case where the negative determination is made, the process proceeds to step S120, while in a case where the positive determination is made, the process proceeds to step S118.

In step S118, the CPU 11 controls the display device 2A to display a line display screen indicating the capturing range specified by the most recent processing of step S110.

In step S120, the CPU 11 determines whether or not the operator has instructed to perform the scanogram capturing by determining whether or not the operation button 2B has been subjected to pressing operation, and returns to step S102 in a case of a negative determination, while proceeds to step S122 in a case of a positive determination.

In step S122, the CPU 11 stops the capturing by the camera 7 and then ends the present information processing.

As described above, in the information processing according to the present embodiment, the update of the line display screen is not performed in a case where the difference D is equal to or less than the threshold value TH, but the present invention is not limited thereto. As described above, in a case where the difference D is equal to or less than the threshold value TH, a form may be adopted, in which the line display screen is updated using the capturing range stored one time before the most recent processing of step S110.

Thereafter, the capturing controller 20 performs the scanogram capturing to acquire the scanogram, and the scanogram is displayed on the display 14. After checking the scanogram, the operator sets the capturing range of the main capturing. Then, the operator performs the pressing operation of the operation button 2B to perform an instruction for the main capturing, the main capturing is performed, so that the three-dimensional CT image of the subject H is acquired.

As described above, with the information processing apparatus 10 according to the present embodiment, image information obtained by optically capturing a subject in a decubitus state on a bed is continuously acquired, control is performed, which is to display a first line in a case where a difference between a position of the first line corresponding to a position of a predetermined part of the subject in an image indicated by the image information at a current point in time and a position of a second line corresponding to a position of the above-described predetermined part of the subject in an image indicated by the image information in a most recent past exceeds a predetermined threshold value. Therefore, as a result in which an update frequency of the line can be reduced, it is possible to suppress the display shake caused by the display of the line indicating the capturing range of the scanogram capturing, as compared with a technology of the related art.

### Second Embodiment

In the present second embodiment, a form example will be described, of which a case where a position obtained by a moving average using images indicated by a plurality of pieces of image information in the most recent past is applied as the position of the second line, unexpected movement of the subject H is also dealt with, and the bed portion 3A of the bed 3 is configured to be movable in an up-down direction.

That is, in the bed 3 according to the present embodiment, the bed portion 3A is movable in the up-down direction under the control of the information processing apparatus 10. However, the present invention is not limited to this form, and the bed portion 3A may be movable in the up-down direction by a manual operation by the operator.

A configuration of the information processing apparatus 10 according to the present embodiment is substantially the same as that according to the first embodiment, except that only a part of the processing by the display controller 24 is different.

The display controller 24 according to the present embodiment applies a position obtained by the moving average using the images indicated by the plurality of pieces of image information in the most recent past as the above-described position of the second line. Here, any number can be applied as the target number of the above-described moving average, but in the present embodiment, the number of frames of 10 in the camera image is applied. As described above, in the present embodiment, a fixed number is applied as the target number of moving averages, but the present disclosure is not limited thereto. For example, a form, in which an input is performed in accordance with a situation of the scanogram capturing, a preference of the operator itself, and the like with respect to the operator, so that the target number of moving averages is appropriately set, may be adopted.

In addition, the display controller 24 according to the present embodiment performs processing (hereinafter, referred to as "second information processing") of executing control using the difference D in a case where the subject H moves by a predetermined amount of movement or greater in order to respond to the unexpected movement of the subject H.

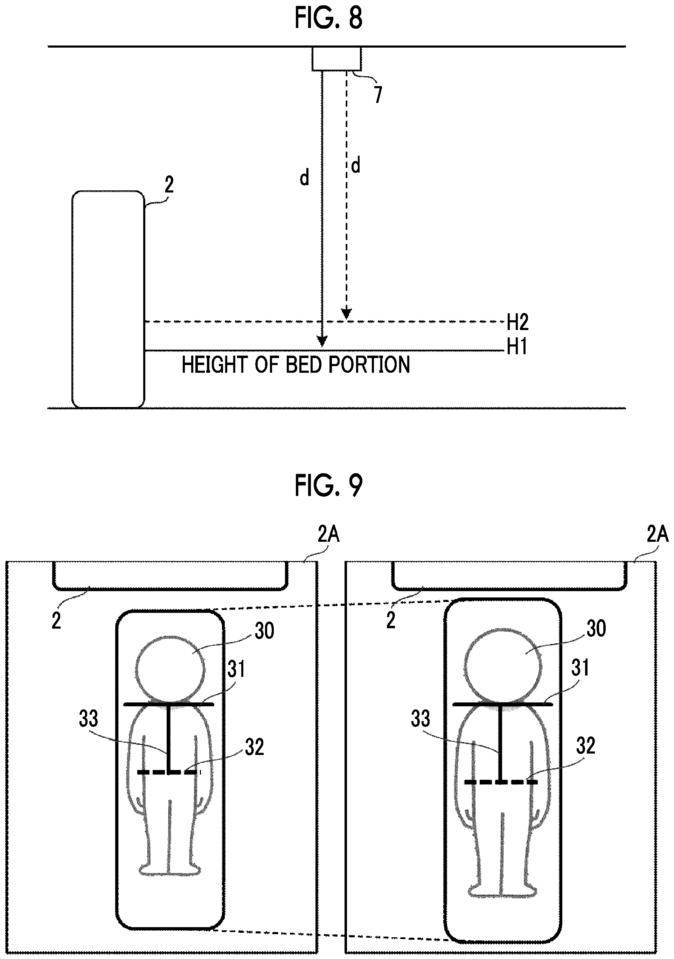

On the other hand, in the present embodiment, since the bed portion 3A of the bed 3 can be moved in the up-down direction, a distance d between the camera 7 and the bed portion 3A of the bed 3 may change as shown in Fig. 8 as an example. In this case, as shown in Fig.9 as an example, a size of a subject image 30 included in the camera image captured by the camera 7 increases as a height of the bed portion 3A of the bed 3 increases. In the example shown in Fig. 9, the left diagram is an example of the camera image in a case where the height of the bed portion 3A in Fig. 8 is a height H1, and the right diagram is an example of the camera image in a case where the height of the bed portion 3A in Fig. 8 is a height H2. Therefore, since a distance per pixel of the camera image is also changed, the difference D is more likely to exceed the threshold value TH as the height of the bed portion 3A increases.

Therefore, in the display controller 24 according to the present embodiment, in a case where the distance between the bed portion 3A and the camera 7 changes as the bed portion 3A of the bed 3 is moved, exceeding a predetermined distance, processing of executing control using the above-described difference D (hereinafter, referred to as "third information processing") is performed. In addition, in a case where the third information processing is executed, the threshold value TH is changed in accordance with the distance d since the difference D is likely to exceed the threshold value TH as described above.

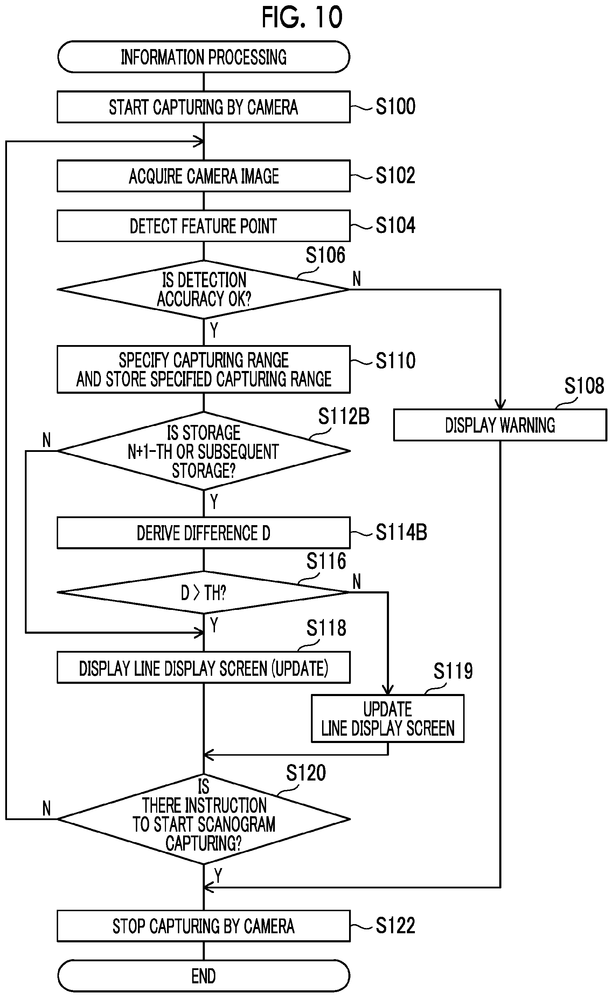

Next, an action of the information processing apparatus 10 according to the present embodiment in a case where the information processing is executed will be described with reference to Fig. 10. Fig. 10 is a flowchart showing a flow of information processing performed in the present embodiment, and in a step in which the same processing as the information processing shown in Fig. 7 is performed, the same step numbers as those in Fig. 7 are assigned, and the description thereof will not be shown.

In step S112B of Fig. 10, the CPU 11 determines whether or not the storage of the capturing range by the processing of step S110 is the N+1-th or subsequent storage, proceeds to step S118 in a case where the negative determination is made while proceeding to step S114B in a case where the positive determination is made.

In step S114B, the CPU 11 derives a difference between the position of the second line obtained by the moving average described above and the position of the first line as the difference D.

In step S119, the CPU 11 applies the position of the second line derived in association with the derivation of the difference D in the immediately preceding processing of step S114B as the position of each line indicating the capturing range, and controls the display device 2A to display the line display screen.

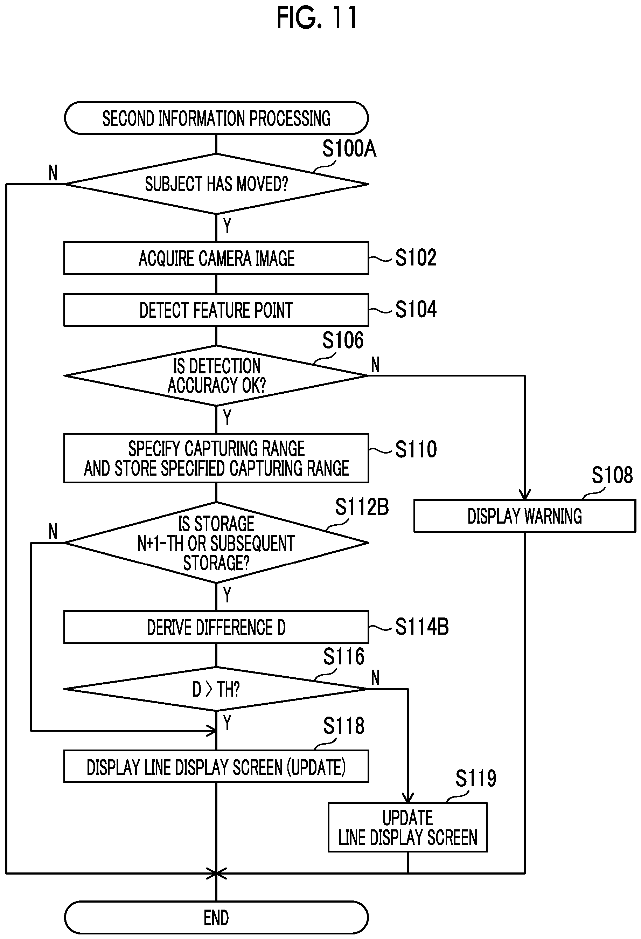

Next, an action of the information processing apparatus 10 according to the present embodiment in a case where the second information processing is executed will be described with reference to Fig. 11. Fig. 11 is a flowchart showing a flow of the second information processing performed in the present embodiment, and in a step in which the same processing as the information processing shown in Fig. 10 is performed, the same step numbers as those in Fig. 10 are assigned, and the description thereof will not be shown. The second information processing according to the present embodiment is executed for each predetermined time (0.1 seconds in the present embodiment).

As shown in Fig. 11, the second information processing according to the present embodiment is different from the information processing according to the present embodiment in only a point that the processing of step S100A is added while the processing of step S102 to step S119 is the same.

That is, in step S100A of Fig. 11, the CPU 11 determines whether or not it is detected that the subject H has moved by a predetermined movement threshold value or more, and in a case where the negative determination is made, ends the second information processing, while in a case where the positive determination is made, and the process proceeds to step S102. In the present embodiment, the determination whether or not the subject H has moved by the predetermined movement threshold value or more is performed by determining whether or not the amount of the two-dimensional movement of the feature points detected by the feature point detection unit 22 between temporally adjacent frames in the camera image is equal to or greater than a predetermined amount. However, it is needless to say that the present invention is not limited thereto.

With the second information processing, it is possible to deal with a case where the unexpected movement occurs in the subject H.

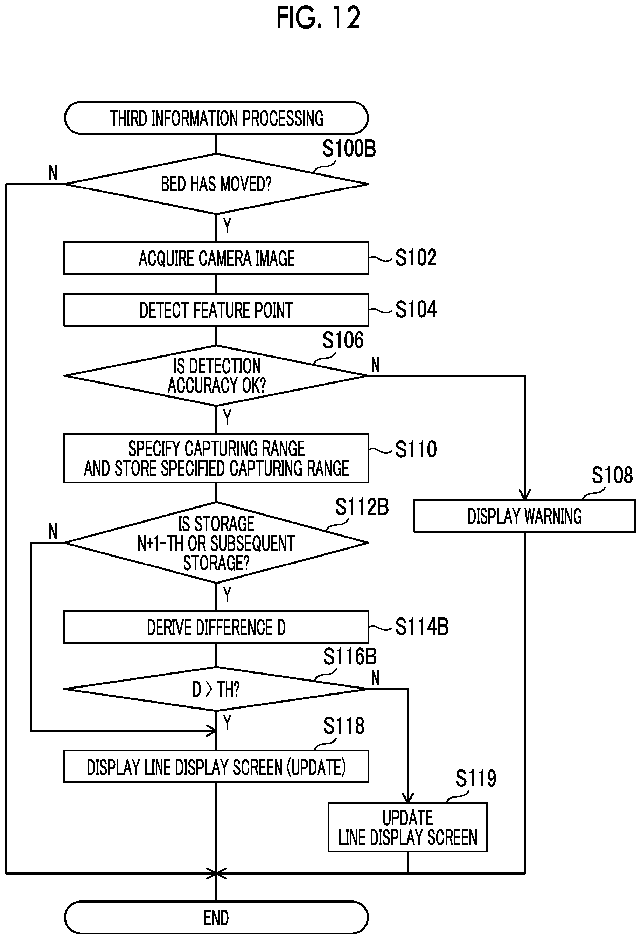

Next, an action of the information processing apparatus 10 according to the present embodiment in a case where the third information processing is executed will be described with reference to Fig. 12. Fig. 12 is a flowchart showing a flow of the third information processing performed in the present embodiment, and in a step in which the same processing as the information processing shown in Fig. 10 is performed, the same step numbers as those in Fig. 10 are assigned, and the description thereof will not be shown. The third information processing according to the present embodiment is also executed for each predetermined time

(0. 1 seconds in the present embodiment).

As shown in Fig. 12, the third information processing according to the present embodiment is different from the information processing according to the present embodiment in only a point that the processing of step S102 to step S119 except for step S116 is the same, while step S116 is set as step S116B and the processing of step S100B is added.

That is, in step S100B of Fig. 12, the CPU 11 determines whether or not the height of the bed portion 3A of the bed 3 has moved exceeding the predetermined distance, and in a case where the negative determination is made, the CPU 11 ends the third information processing, while in a case where the positive determination is made, the process proceeds to step S102.

Then, in step S116B, the CPU 11 applies a predetermined threshold value as the threshold value TH in accordance with the height of the bed portion 3A of the bed 3, and determines whether or not the difference D exceeds the threshold value TH. In a case where the negative determination is made, the process proceeds to step S119, while in a case where the positive determination is made, the process proceeds to step S118.

With the third information processing, it is possible to accurately respond even in a case where the height of the bed portion 3A of the bed 3 is changed.

As described above, with the information processing apparatus 10 according to the present embodiment, the position obtained by the moving average using the images indicated by the plurality of pieces of image information in the most recent past is applied as the position of the second line. Accordingly, as a result of being able to cope with a case where the camera image varies between the frames, it is possible to more effectively suppress the display shake caused by the display of the line indicating the capturing range of the scanogram capturing.

In addition, with the information processing apparatus 10 according to the present embodiment, in a case where the bed portion 3A of the bed 3 is movable in the up-down direction and the camera 7 is fixed above the bed 3, the control using the difference D is performed in a case where the distance between the bed portion 3A and the camera 7 changes as the bed portion 3A is moved, exceeding the predetermined distance. Therefore, even in a case where the bed portion 3A is moved in the up-down direction, the above-described display shake can be more accurately suppressed.

Further, with the information processing apparatus 10 according to the present embodiment, the threshold value TH is changed in accordance with the distance d. Therefore, even in a case where the bed portion 3A is moved in the up-down direction, the above-described display shake can be more accurately suppressed.

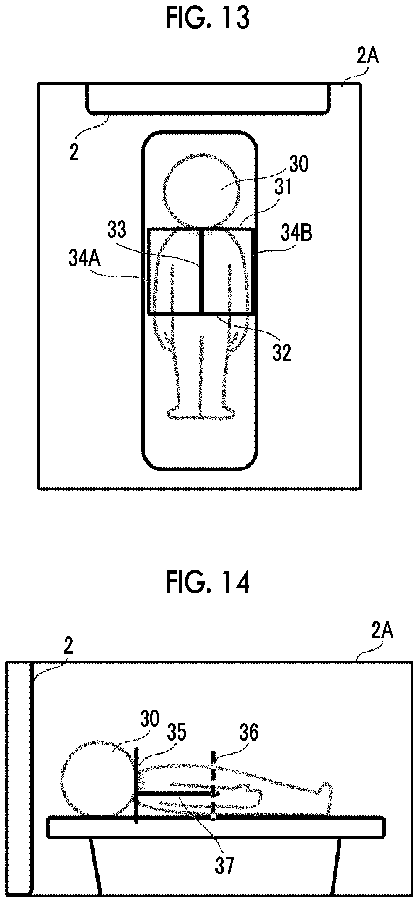

The line display screen applied in each of the above-described embodiments is an example and is not limited to the one shown in drawings. For example, as shown in Fig. 13 as an example, a form may be adopted in which lines 34A and 34B indicating the range in the left-right direction in the capturing range of the scanogram capturing are also displayed on the line display screen.

In addition, in each of the above-described embodiments, a case where the capturing range in a case where the subject H is viewed from above is applied as the capturing range of the scanogram capturing has been described, but the present invention is not limited thereto. For example, a form may be adopted in which the capturing range in a case where the subject H is viewed from the side is applied as the capturing range of the scanogram capturing. In this form, the camera 7 is also provided on the side of the subject H, or the above-described NIR camera is applied as the camera 7.

Fig. 14 shows an example of a line display screen in this form. In Fig. 14, the scanogram start line is a line 35, the scanogram end line is a line 36, and the midline is a line 37.

In addition, a type of lines shown in the above-described form example is not limited to a solid line or a broken line shown in the drawing, and other types such as a one-dot chain line and a two-dot chain line may be applied. Further, from the viewpoint of ease of visual recognition, a form may be adopted in which a color or a thickness of the line, a display state such as blink display, reverse display, or the like can be changed for each use of each line.

In addition, in each of the above-described embodiments, the information processing apparatus according to the present disclosure is applied to the CT device, but the present disclosure is not limited thereto. The information processing apparatus according to the present disclosure may be applied to an MRI device or the like as long as a capturing apparatus is for acquiring a scanogram for setting a capturing range before main capturing.

In addition, in each of the above-described embodiments, the information processing apparatus comprises the capturing controller 20, but the present invention is not limited thereto. The capturing controller 20 may be provided separately from the information processing apparatus.

In addition, in the above-described embodiment, for example, the following various processors can be used as a hardware structure of processing units that execute various types of processing, such as the capturing controller 20, the camera controller 21, the feature point detection unit 22, the capturing range specifying unit 23, and the display controller 24. As described above, the above-described various processors include a programmable logic device (PLD) as a processor of which the circuit configuration can be changed after manufacture, such as a field-programmable gate array (FPGA), a dedicated electrical circuit as a processor having a dedicated circuit configuration for executing specific processing such as an application-specific integrated circuit (ASIC), and the like, in addition to the CPU as a general-purpose processor that functions as various processing units by executing software (program).

One processing unit may be configured by one of the various processors, or may be configured by a combination of the same or different types of two or more processors (for example, a combination of a plurality of FPGAs or a combination of the CPU and the FPGA). Further, a plurality of processing units may be configured with one processor.

As an example where a plurality of processing units are configured by one processor, first, there is a form in which one processor is configured by a combination of one or more CPUs and software as typified by a computer, such as a client or a server, and this processor functions as a plurality of processing units. Second, as represented by a system-on-chip (SoC) or the like, there is a form of using a processor for realizing the function of the entire system including a plurality of processing units with one integrated circuit (IC) chip. As described above, the various types of processing units are configured using one or more of the above-described various types of processors as a hardware structure.

Furthermore, as the hardware structure of the various processors, more specifically, an electrical circuit (circuitry) in which circuit elements such as semiconductor elements are combined can be used.

From the above description, the invention described in the following appendixes can be understood.

### Appendix 1

An information processing apparatus comprising at least one processor,
wherein the processor is configured to:
continuously acquire image information obtained by optically capturing a subject in a decubitus state on a bed; and
perform control to display a first line in a case where a difference between a position of the first line corresponding to a position of a predetermined part of the subject in an image indicated by the image information at a current point in time and a position of a second line corresponding to a position of the predetermined part of the subject in an image indicated by the image information in a most recent past exceeds a predetermined threshold value.

### Appendix 2

The information processing apparatus according to appendix 1,
wherein the processor is configured to perform control to display the second line in a case where the difference is equal to or less than the threshold value.

### Appendix 3

The information processing apparatus according to appendix 1 or 2,
wherein the position of the second line is a position obtained by a moving average using images indicated by a plurality of pieces of the image information in the most recent past.

### Appendix 4

The information processing apparatus according to appendix 1 or 2,
wherein the position of the second line is a position obtained by an image indicated by a single piece of the image information in the most recent past.

### Appendix 5

The information processing apparatus according to any one of appendixes 1 to 4,
wherein the processor is configured to perform control using the difference in a case where a distance between the bed and a capturing apparatus that performs the optical capturing changes exceeding a predetermined distance due to movement of the bed, in a case where the bed is movable in an up-down direction and the capturing apparatus is fixed above the bed.

### Appendix 6

The information processing apparatus according to appendix 5,
wherein the processor changes the threshold value in accordance with the distance.

### Appendix 7

A medical image capturing apparatus comprising:
the information processing apparatus according to any one of appendixes 1 to 6; and
a radiography apparatus controlled by the information processing apparatus.

### Appendix 8

An information processing method causing a computer to execute:
continuously acquiring image information obtained by optically capturing a subject in a decubitus state on a bed; and
performing control to display a first line in a case where a difference between a position of the first line corresponding to a position of a predetermined part of the subject in an image indicated by the image information at a current point in time and a position of a second line corresponding to a position of the predetermined part of the subject in an image indicated by the image information in a most recent past exceeds a predetermined threshold value.

### Appendix 9

A program causing a computer to execute a process comprising:
continuously acquiring image information obtained by optically capturing a subject in a decubitus state on a bed; and
performing control to display a first line in a case where a difference between a position of the first line corresponding to a position of a predetermined part of the subject in an image indicated by the image information at a current point in time and a position of a second line corresponding to a position of the predetermined part of the subject in an image indicated by the image information in a most recent past exceeds a predetermined threshold value.

## Claims

1. An information processing apparatus (10) comprising a processor (11),
wherein the processor (11) is configured to:
continuously acquire image information obtained by optically capturing a subject (H) in a decubitus state on a bed (3); and
perform control to display a first line in a case where a difference (D) between a position of the first line corresponding to a position of a predetermined part of the subject in an image indicated by the image information at a current point in time and a position of a second line corresponding to a position of the predetermined part of the subject (H) in an image indicated by the image information in a most recent past exceeds a predetermined threshold value (TH).

2. The information processing apparatus (10) according to claim 1,
wherein the processor (11) is configured to perform control to display the second line in a case where the difference (D) is equal to or less than the threshold value (TH).

3. The information processing apparatus (10) according to claim 1 or 2,
wherein the position of the second line is a position obtained by a moving average using images indicated by a plurality of pieces of the image information in the most recent past.

4. The information processing apparatus (10) according to claim 1 or 2,
wherein the position of the second line is a position obtained by an image indicated by a single piece of the image information in the most recent past.

5. The information processing apparatus (10) according to claim 1,
wherein the processor (11) is configured to perform control using the difference (D) in a case where a distance (d) between the bed (3) and a capturing apparatus (7) that performs the optical capturing changes exceeding a predetermined distance due to movement of the bed (3), in a case where the bed (3) is movable in an up-down direction and the capturing apparatus (7) is fixed above the bed (3).

6. The information processing apparatus (10) according to claim 5,
wherein the processor (11) is configured to change the threshold value (TH) in accordance with the distance (d).

7. A medical image capturing apparatus (2, 3, and 4) comprising:
the information processing apparatus (10) according to claim 1; and
a radiography apparatus (2 and 3) controlled by the information processing apparatus (10).

8. An information processing method causing a computer (10) to execute:
continuously acquiring image information obtained by optically capturing a subject (H) in a decubitus state on a bed (3); and
performing control to display a first line in a case where a difference (D) between a position of the first line corresponding to a position of a predetermined part of the subject (H) in an image indicated by the image information at a current point in time and a position of a second line corresponding to a position of the predetermined part of the subject (H) in an image indicated by the image information in a most recent past exceeds a predetermined threshold value (TH).

9. A storage medium (13) storing a program (12) executable by a computer (10) to execute a process comprising:
continuously acquiring image information obtained by optically capturing a subject (H) in a decubitus state on a bed (3); and
performing control to display a first line in a case where a difference (D) between a position of the first line corresponding to a position of a predetermined part of the subject (H) in an image indicated by the image information at a current point in time and a position of a second line corresponding to a position of the predetermined part of the subject (H) in an image indicated by the image information in a most recent past exceeds a predetermined threshold value (TH).
